# FASCICULE DE BREVET EUROPEEN

(11) **EP 1 562 500 B1**
(45) Date de publication et mention de la délivrance du brevet: **28.03.2012**
(21) Numéro de dépôt: 03778377.6
(22) Date de dépôt: 31.07.2003
(51) Int. Cl.: A61B 17/70

(54) **DISPOSITIF DE DISTRACTION ET D'AMORTISSEMENT AJUSTABLE A LA CROISSANCE DU RACHIS**
DISTRAKTIONS- UND DäMPFUNGSSYSTEM, DAS BEI WACHSTUM DER WIRBELSäULE VERSTELLBAR IST
DISTRACTION AND DAMPING SYSTEM WHICH CAN BE ADJUSTED AS THE VERTEBRAL COLUMN GROWS

(30) Priorité: 13.08.2002 FR 0210248
(43) Date de publication de la demande: 17.08.2005
(73) Titulaire: Siguler Guff Distressed Opportunities Fund III, LP, New York, NY 10022 (US)
(72) Inventeur: FORTIN, Frédéric, F-33600 Pessac (FR); ZELLER, Reinhard, F-92100 Boulogne (FR); DIMEGLIO, Alain, F-34090 Montpellier (FR)
(74) Mandataire: Molnia, David
(86) Numéro de dépôt international: PCT/FR2003/002435
(87) Numéro de publication internationale: WO 2004/016185

(56) Documents cités:
- EP-A2- 0 677 277
- FR-A- 2 794 357
- FR-A- 2 814 936
- US-B1- 6 402 750

## Description

### DOMAINE DE L'INVENTION

L'invention concerne un dispositif de distraction et de soutien réglable, implanté sur le tronc d'un enfant et constitué de la combinaison de plusieurs dispositifs permettant de redresser, de soutenir, d'amortir les sollicitations mécaniques, et pouvant être réajusté en fonction de la croissance de l'enfant; ces fonctions ne pouvant être obtenues par chacun des dispositifs pris séparément

### ART ANTERIEUR :

La demande de brevet Fr-A-2794357 (*distracteur costal*..) décrit et revendique un dispositif mécanique qui est capable de suivre la croissance des os dans une malformation en corrigeant les déformations du tronc sans interdire ni bloquer la croissance du patient, mais il est limité dans son application à une déformation au niveau du thorax, il ne peut s'appliquer à une déformation impliquant le rachis lombaire de l'enfant car on risque d'obtenir un blocage de la croissance en fixant le dispositif directement sur les vertèbres. Le préambule de la revendication 1 est basé sur ce dispositif.

La demande de brevet FR-A-2827498. *Dispositif de liaison vertébral souple constitué d'éléments palliant une déficience* deposée avant la date de priorité du present brevet est essentiellement un amortisseur constitué d'éléments rigides enserrant des moyens viscoélastiques qui vont amortir les sollicitations mécaniques dans les directions souhaitées.

Ce dispositif a été conçu pour résister aux sollicitations subies par les vertèbres du corps humain, ceci de manière multiaxiale, il n'a pas été conçu pour s'allonger lors des déplacements en extension comme c'est le cas du dispositif décrit dans le précédent brevet.

La demande de brevet FR-A-2814936 est similaire mais elle divulgue des moyens élastiques au lieu des moyens viscoélastiques.

On connaît du document EP-A-0677277 un ensemble prothétique rachidien qui est rempli d'un produit viscoélastique d'amortissement. Le dispositif de la présente invention permet:
- de stabiliser la cage thoracique en autorisant une développement des poumons.
- de redresser une colonne vertébrale complète en croissance sans blocage des vertèbres
- d'amortir les sollicitations mécaniques externes
- de disposer d' un système de réglage facilement accessible ne nécessitant aucune intervention lourde ou invasive

Les ancrages du premier dispositif résultant de la première invention (FR-A-2794357) sont réalisés par des étriers encerclant les os; ce premier dispositif convenant parfaitement aux fixations du thorax induirait cependant des contraintes importantes dans les moyens de fixation utilisés pour les vertèbres lombaires, ce qui entraînerait un risque de rupture notamment de la vis de fixation, ce qui n'est bien sur pas admissible.

La présente invention résout ce problème grâce à son aptitude à régler les problèmes induits par les grandes déformations en incluant le rachis lombaire.

Dans le rapport, de recherche de la demande de brevet français dont le present brevet prend sa priorité (dépôt 0210248) trois documents antérieurs sont opposés 1- brevet Atkinson (classé X) US 6402 750 B 1

Le seul moyen comparable dans ce document est le piston qui dans cette invention ne peut avoir qu'un déplacement axial , il n'existe aucune possibilité de suivre un mouvement curviligne en phase avec la forme du rachis. Pour régler ce dispositif on est obligé de procéder à un démontage complet d'ou il s'en suit une intervention lourde ! ce qui n'est aucunement le cas de notre invention qui se contente d'une intervention légère sous anesthésie locale pour régler un moyen central ,dont l'accès ne nécessite qu'une petite incision sous la peau.

2- Les brevets Fortins FR 2794357 et FR 2814936 qui concernent au départ deux dispositifs conçus de manière indépendante et dont la combinaison sans modification serait impossible .

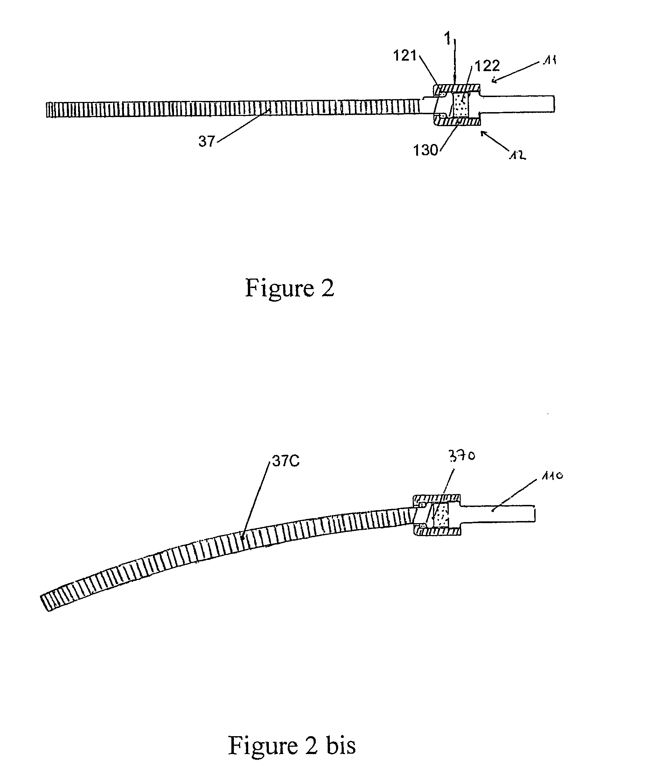

Le résultat obtenu n'était nullement garanti; il a été rendu possible grâce à la création et à l'implantation de moyens nouveaux telles que les tiges à denture 37 et 37c (fig 2 et 2bis) pouvant être cintrées à la demande et dont une des extrémités à la forme d'un piston 370.

Ce résultat inattendu a été le bon fonctionnement du dispositif, ce qui prouve que le fonctionnement de cette invention combinaison des deux dispositifs antérieurs a été réalisé, grâce à de nombreux essais effectués avec différents rayons de courbure pour les tiges, caractéristiques qui se distinguent de l'art antérieur.

Les dessins permettant de mieux comprendre l'invention sont :

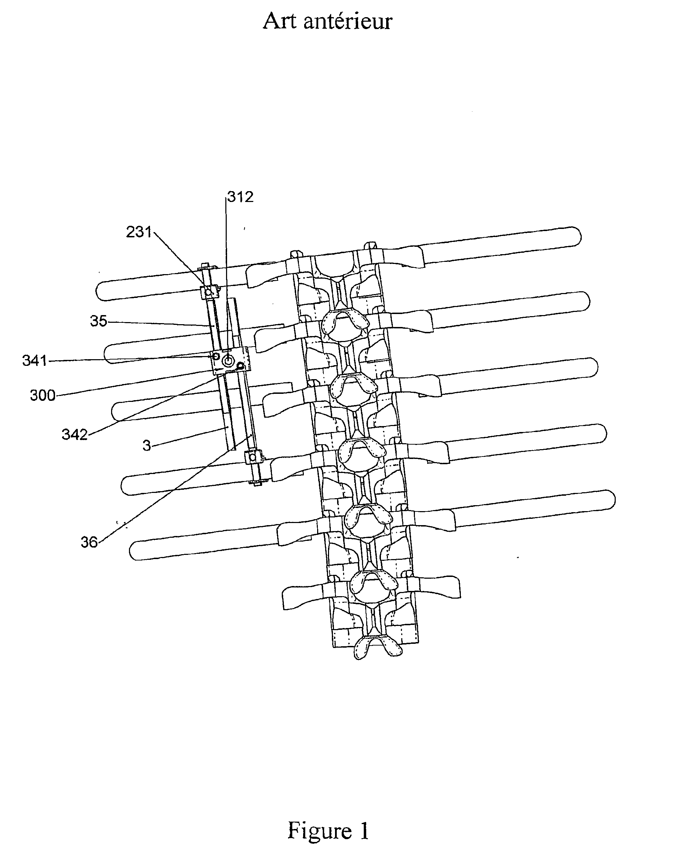

Fig 1 planche 1/9 : dispositif de distraction pour les os d'enfant (art antérieur)
Fig 2 et 2bis de la planche 2/9: dispositif de liaison souple et amortissant comportant 2 tiges dont une comporte une denture pour servir de crémaillère (tiges rectilignes ou courbées)
Fig 3 planche 3/9 : premier exemple d'un dispositif (objet de l'invention) combinant le dispositif de distraction pour les os d'enfant avec le dispositif de liaison souple et amortissant.
Fig 4 planche 3/9 : deuxième exemple du dispositif objet de l'invention combinant le dispositif de distraction avec deux dispositifs de liaison souple et amortissant.

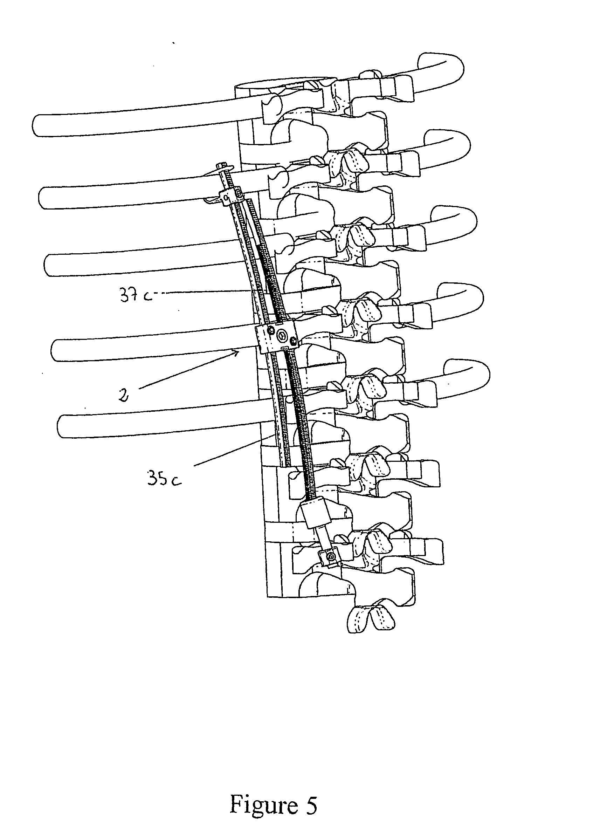

Fig 5 planche 4/9 :dispositif objet de l'invention combinant les deux dispositifs placé sur une côte et sur une vertèbre et comportant deux tiges de distraction curvilignes
Fig 6 planche 5/9 autre exemple du dispositif objet de l'invention mis en place sur une côte et une vertèbre et comportant des tiges de distraction rectilignes et curvilignes.
Fig 7 planche 5/9 dispositif objet de l'invention travaillant avec des tiges rectilignes

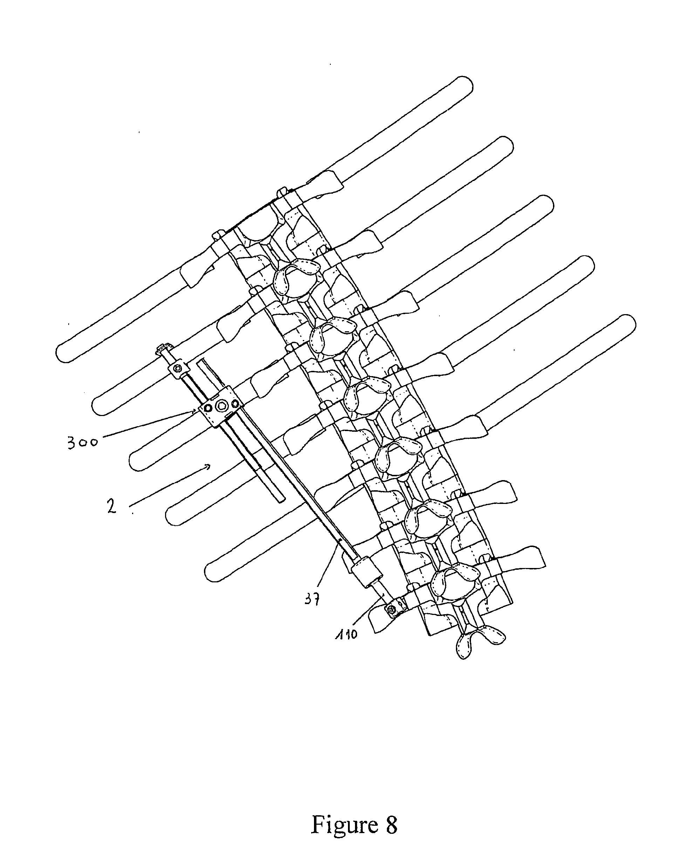

Fig 8 planche 6/9 Mise en place du dispositif objet de l'invention sur un tronc déformé
Fig 9 planche 7/9 visualisation du dispositif objet de l'invention après redressement du tronc initialement déformé, l'amortisseur se positionnant en travers sous l'influence des efforts de redressement
Fig 10 planche 8/9 visualisation du dispositif combiné, l'amortisseur étant moins contraint suite à la croissance de l'enfant
. Fig 11 planche 9/9 positionnement de plusieurs dispositifs sur un tronc permettant de contrôler et de le redresser entièrement

### DESCRIPTION

Le Dispositif 2 combinant les dispositifs 3 et 1 est constitué d'un dispositif 3 dénommé distracteur pour les os d'enfants d'un dispositif 1 dénommé amortisseur des sollicitations mécaniques.

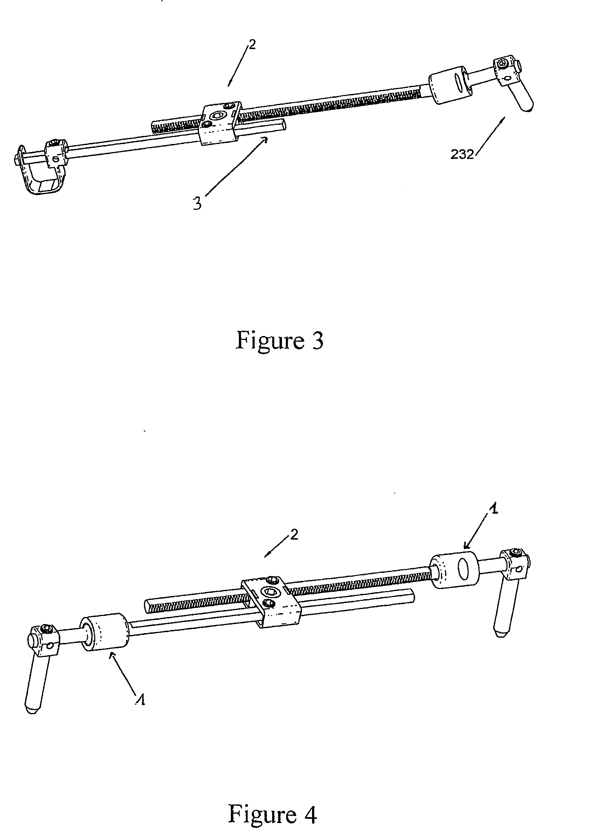

Le dispositif 2(fig 3) ainsi formé permet donc à la fois :
- de redresser la colonne vertébrale et de de stabiliser le tronc d'un enfant
- de suivre sa croissance et d'allonger les distances d'accrochages par des interventions minimes
- d'alléger les charges mécaniques appliquées au moyens d'accrochage
- d'amortir les sollicitations mécaniques

Nous rappellerons brièvement pour mieux comprendre l'invention les moyens utilisés dans les dispositifs 3 et 1

Le dispositif 3 permet de gérer l'évolution de la déformation du tronc d'un enfant; il est implantable facilement dans le corps humain , grâce à son faible encombrement; il comporte :
deux tiges 35 et 36 à denture montées sur un moyen central 300 pourvu d'un trou 312 dans lequel on peut engager un outil pour régler la distance des moyens d'accrochages 231 aux os, on bloque le dispositif dans une position déterminée par serrage de 2 vis 341 et 342 situées sur le moyen central 300.

Dans l'art antérieur les tiges 35 et 36 étaient rectilignes ,elles pouvaient être courbées à leur extrémités pour faciliter la mise en place des moyens d'accrochages, alors que le présent dispositif 2 peut comporter des tiges cintrées ou courbées 35c et 36c, ceci dans toute leur longueur. Dans ce cas des résultats d'essai ont montré un fonctionnement normal du dispositif. L'avantage procuré par cette amélioration est d'éviter de blesser les tissus environnant et d'allonger les distances d'accrochage en suivant parfaitement une courbure choisie au départ par l'opérateur.

De plus cette courbure peut être modifiée grâce à la malléabilité du matériau qui constitue les tiges qui après essai peuvent accepter d'être déformées par l'opérateur.

Le dispositif de liaison intervertébrale souple 1 est lui-même constitué de deux ensembles de moyens:

Un premier ensemble de moyens 11 composé de moyens rigides 110,130,37 fabriqués en matériaux biocompatibles assurant une bonne tenue mécanique du dispositif en transmettant intégralement les efforts sans se déformer.

Un deuxième ensemble de moyens 12 formé de moyens souples et amortissant 121 et 122 fabriqués en matériaux viscoélastiques biocompatibles, acceptant les déformations élastiques répétées, la combinaison de ces deux ensembles de moyens permettant de résister et d'amortir les sollicitations mécaniques auxquelles il sera soumis, pour palier à toute déficience des liaisons du rachis.

Chacun des dispositifs 3 et 1 ont des moyens qui sont compatibles, par leur conception et fonctions principales auxquels on ajoute des moyens nouveaux pour leur permettre de se combiner.

Comme nous l'avons déjà mentionné, le dispositif 3 peut recevoir des tiges rectilignes , ou curvilignes , courbées selon des rayons différents. On peut mettre en place un dispositif 2 composé d'un dispositif 3 ayant
d'un coté une tige rectiligne 37
de l'autre une tige courbée ou cintrée 36 c

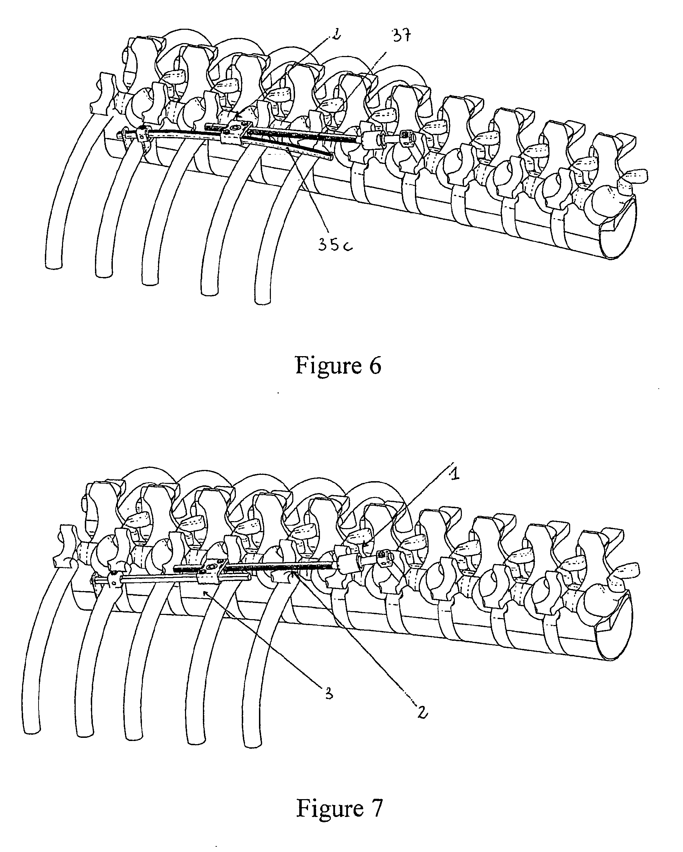

Dans ce dernier cas 36c est courbée pour ne pas blesser et pour permettre un allongement proche de la courbure anatomique (figure 6). Le Dispositif 2 résultant de la combinaison entre 3 et 1 permet de redresser le tronc par réglage du moyen central 300 et de ses moyens 312, 341 et 342 par une intervention très peu invasive, très ciblée effectuée sous anesthésie locale

Le dispositif de distraction 2 comporte au moins une tige 37 ou 37c rectiligne ou curviligne à denture, dont une des extrémité comporte un plateau cylindrique 370 s'intégrant parfaitement dans les moyens viscolélastiques 121 et 122 et rigide 130 du dispositif 1, ce qui permet au dispositif de distraction 2 d'être amorti et de pouvoir s'accrocher à une vertèbre ,alors qu'il est au départ désaligné vis à vis du dispositif de distraction 3

On redresse ainsi le tronc par le moyen central 300 que l'on vient ensuite bloquer, par les vis 341 et 342 , par une petite clef à six pans, introduite dans la vis 312 par un petit orifice ne nécessitant qu'une intervention minime. Le dispositif 2 va alors remplir sa fonction de soutien du tronc redressé.

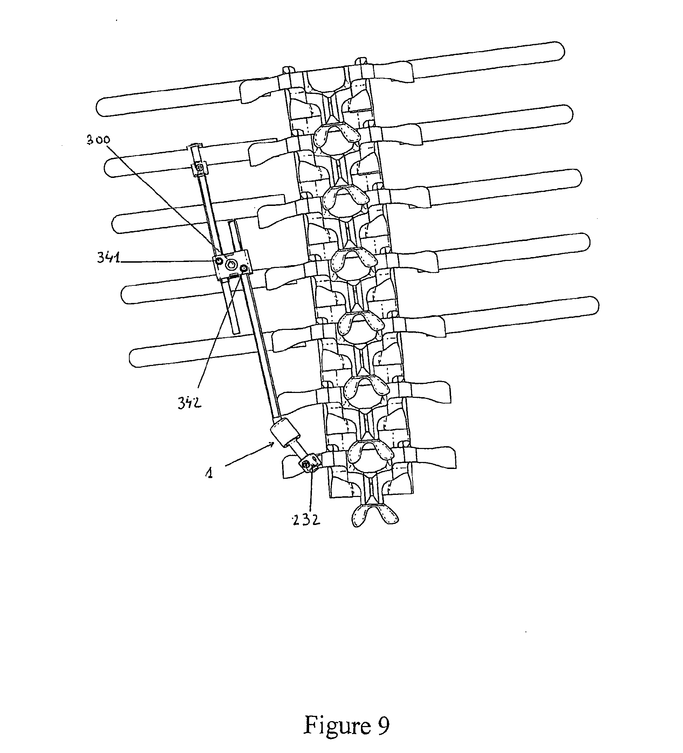

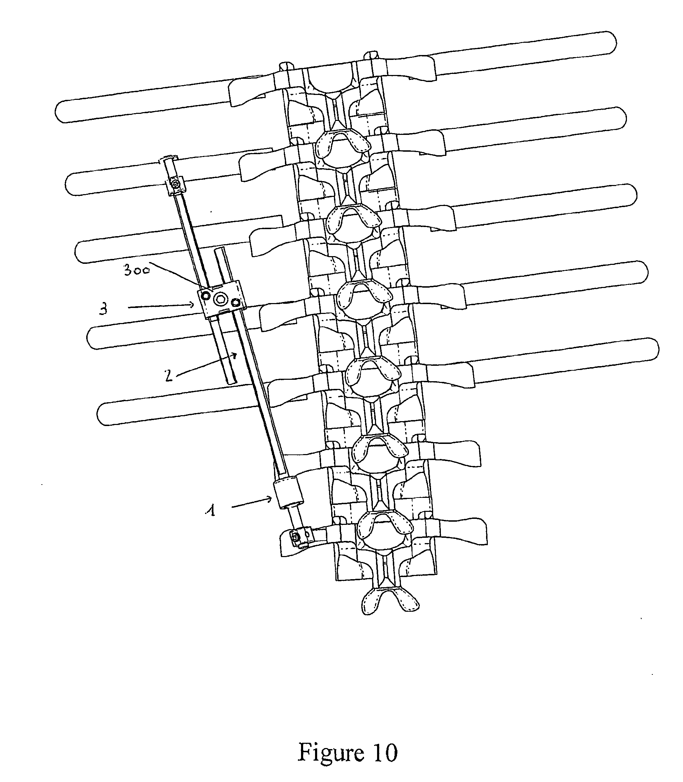

Après croissance , on visionne le dispositif 2 notamment l'alignement de la tige 37 avec la tige 110; l'opérateur peut alors intervenir sur le moyen 300 dans le but de redresser à nouveau le tronc. Suite aux efforts de redressement le dispositif amortisseur 1 faisant partie du dispositif 2 se place légèrement en travers (figure 9) il sert ainsi d'indicateur de croissance et d'effort pour déterminer en fonction de chaque cas la nécessité du redressement. Dans le cas ou l'on pose un dispositif 2 sur le tronc d'un enfant en début croissance, l'amortisseur 1 peut être placé entre une vis de fixation à une vertèbre et la tige 37 conçue pour la liaison avec le dispositif 3 (fig 9 moyen 232) . Ce dispositif 2 va autoriser et permettre de suivre la croissance de l'enfant tout en évitant des contraintes importantes dans la vis d'accrochage 232 (figure 10); les moyens viscoélastiques 121 et 122 en absorbant les chocs et les sollicitations dynamiques dans lesdites vis à os 232 vont éviter les ruptures; le passage à la position :"alignement" des tiges 110 avec 37 ou 37c est un indicateur fiable.

Si les deux tiges sont alignées , il est nécessaire de remettre le dispositif en tension , car les contraintes appliquées au dispositif 1 ont diminué de sorte que les deux tiges sont revenues dans le même axe grâce à l'élasticité du dispositif 1 et à la croissance de l'enfant

En plus des limitations des contraintes exercées sur les vis après redressement du tronc, le dispositif 1 amortit les sollicitations mécaniques extérieures et permet grâce à sa souplesse de ne pas gêner les mouvements vertébraux.
ces différentes opérations de redressement et de stabilisation du tronc d'un enfant peuvent être facilement répétées; le dispositif 2 peut être ainsi réajusté autant de fois que nécessaire par une action très localisée sur le moyen 300

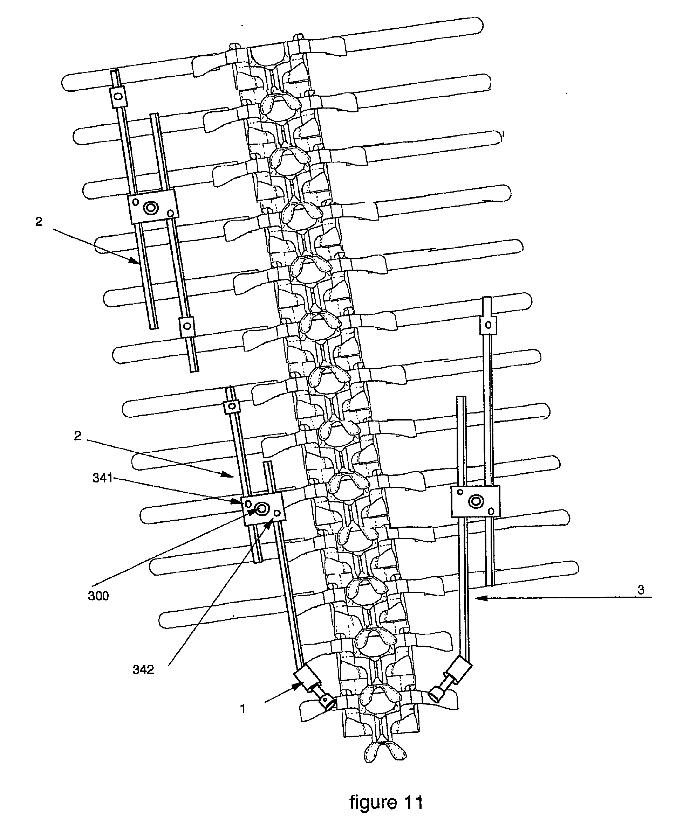

En outre la multiplicité des combinaisons des moyens 1 et 3 permet de placer plusieurs dispositif 2 sur la colonne vertébrale (figure 11) pour suivre la croissance et redresser le tronc.

## Revendications

1. Dispositif de distraction et d'amortissement comprenant
une première tige (37) avant un premier bout;
une deuxième tige (35) avant un deuxième bout; et
un coupleur mécanique (300) connectant la première tige avec la deuxième tige et permettant de régler les positions de la première tige et de la deuxième tige en sorte que la distance entre le premier bout et le deuxième bout peut être contrôler pour redresser la colonne vertébrale d'un patient; **caracterisé en ce qu'**il comprend en outre:
un premier élément d'amortissement qui comprend
un cylindre rigide (130),
un premier élément viscoélastique (121) disposé dans le cylindre rigide; et
un deuxième élément viscoélastique (122) disposé dans le cylindre rigide, le premier bout de la première tige comprenant une partie distal (370) en forme d'une tête de piston disposée entre le premier élément élastique et le deuxième élément élastique pour faciliter amortissement pendant la tension et la compression.

2. Dispositif selon la revendication 1 **caractérisé en ce que** la première tige et la deuxième tige sont dentées.

3. Dispositif selon l'une quelconque des précédentes revendications **caractérisé en ce que** l'une des la première tige et la deuxième tige est rectiligne et l'autre des la première tige et la deuxième tige est curviligne.

4. Dispositif selon l'une quelconque des revendications 1 - 2 **caractérisé en ce que** la première tige et la deuxième tige sont curviligne.

5. Dispositif selon l'une quelconque des précédentes revendications **caractérisé en ce que** le deuxième bout comprend des moyens d'accrochages configurés pour accrocher une côte.

6. Dispositif selon l'une quelconque des précédentes revendications comprenant
un deuxième élément d'amortissement comprenant
un cylindre rigide;
un premier élément viscoélastique disposé dans le cylindre rigide; et
un deuxième élément viscoélastique disposé dans le cylindre rigide, le deuxième bout de la deuxième tige comprenant une partie distale en forme d'une tête de piston disposée entre le premier élément élastique du deuxième élément d'amortissement et le deuxième élément élastique du deuxième élément d'amortissement pour faciliter amortissement pendant la tension et la compression.

7. Dispositif selon l'une quelconque des précédentes revendications comprenant une troisième tige connectée aux premier élément d'amortissement et mobile entre une position alignée avec la première tige et une position en travers à la première tige.

## Claims

1. A distraction and damping device comprising a first rod (37) having a first end;
a second rod (35) having a second end; and
a mechanical coupler (300) connecting the first rod to the second rod and allowing adjustment of the positions of the first rod and the second rod such that the distance between the first end and the second end may be controlled to straighten the spinal column of a patient, **characterised in that** it further comprises:
a first damping element, which comprises
a rigid cylinder (130),
a first viscoelastic element (121) disposed in the rigid cylinder; and
a second viscoelastic element (122) disposed in the rigid cylinder, the first end of the first rod comprising a distal part (370) in the form of a piston head disposed between the first elastic element and the second elastic element to facilitate damping during tension and compression.

2. A device according to claim 1, **characterised in that** the first rod and the second rod are toothed.

3. A device according to either one of the preceding claims, **characterised in that** one of the first and second rods is rectilinear and the other of the first and second rods is curvilinear.

4. A device according to either one of claims 1 or 2, **characterised in that** the first rod and the second rod are curvilinear.

5. A device according to any one of the preceding claims, **characterised in that** the second end comprises hooking means configured for hooking onto a rib.

6. A device according to any one of the preceding claims, comprising a second damping element comprising
a rigid cylinder;
a first viscoelastic element disposed in the rigid cylinder; and a
second viscoelastic element disposed in the rigid cylinder, the second end of the second rod comprising a distal part in the form of a piston head disposed between the first elastic element of the second damping element and the second elastic element of the second damping element to facilitate damping during tension and compression.

7. A device according to any one of the preceding claims, comprising a third rod connected to the first damping element and movable between a position in which it is aligned with the first rod and a position across the first rod.

## Patentansprüche

1. Disktraktions- und Dämpfungsvorrichtung, umfassend einen ersten Stab (37) mit einem ersten Ende;
einen zweiten Stab (25) mit einem zweiten Ende und
eine mechanische Kupplung (300), die den ersten Stab mit dem zweiten Stab verbindet und es ermöglicht, die Positionen des ersten Stabs und des zweiten Stabs so einzustellen, dass der Abstand zwischen dem ersten Ende und dem zweiten Ende gesteuert werden kann, um die Wirbelsäule eines Patienten aufzurichten; **dadurch gekennzeichnet, dass** die Vorrichtung außerdem Folgendes umfasst:
ein erstes Dämpfungselement, umfassend
einen starren Zylinder (130);
ein erstes viskoelastisches Element (121), das in dem starren Zylinder angeordnet ist; und
ein zweites viskoelastisches Element (122), das in dem starren Zylinder angeordnet ist, wobei das erste Ende des ersten Stabs einen distalen Teil (370) in der Form eines Kolbenkopfs umfasst, der zwischen dem ersten elastischen Element und dem zweiten elastischen Element angeordnet ist, um die Dämpfung während der Spannung und der Kompression zu erleichtern.

2. Vorrichtung nach Anspruch 1, **dadurch gekennzeichnet, dass** der erste Stab und der zweite Stab gezahnt sind.

3. Vorrichtung nach einem der vorhergehenden Ansprüche, **dadurch gekennzeichnet, dass** entweder der erste Stab oder der zweite Stab geradlinig ist und der jeweils Andere, der zweite Stab oder der erste Stab, krummlinig ist.

4. Vorrichtung nach einem der Ansprüche 1 bis 2, **dadurch gekennzeichnet, dass** der erste Stab und der zweite Stab krummlinig sind.

5. Vorrichtung nach einem der vorhergehenden Ansprüche, **dadurch gekennzeichnet, dass** das zweite Ende Einhängemittel umfasst, die zum Einhängen einer Rippe ausgebildet sind.

6. Vorrichtung nach einem der vorhergehenden Ansprüche, umfassend ein zweites Dämpfungselement, das umfasst
einen starren Zylinder;
ein erstes viskoelastisches Element, das in dem starren Zylinder angeordnet ist; und
ein zweites viskoelastisches Element, das in dem starren Zylinder angeordnet ist, wobei das zweite Ende des zweiten Stabs einen distalen Teil in der Form eine Kolbenkopfs umfasst, der zwischen dem ersten elastischen Element des zweiten Dämpfungselements und dem zweiten elastischen Element des zweiten Dämpfungselements angeordnet ist, um die Dämpfung während der Spannung und der Kompression zu erleichtern.

7. Vorrichtung nach einem der vorhergehenden Ansprüche, umfassend einen dritten Stab, der mit dem ersten Dämpfungselement verbunden ist und zwischen einer mit dem ersten Stab ausgerichteten Position und einer Position quer zum ersten Stab beweglich ist.
